# EUROPEAN PATENT APPLICATION

(11) **EP 3 892 297 A1**
(43) Date of publication of application: **13.10.2021**
(21) Application number: 20169047.6
(22) Date of filing: 09.04.2020
(51) Int. Cl.: A61K 39/215, C07K 16/10, C07K 14/005

(54) **PEPTIDES AND COMBINATIONS OF PEPTIDES FOR USE IN IMMUNOTHERAPY AGAINST AN INFECTION BY SARS-COV-2 (COVID-19)**

(71) Applicant: Eberhard Karls Universität Tübingen Medizinische Fakultät, 72074 Tübingen (DE)
(72) Inventor: Walz, Juliane, 72074 Tübingen (DE); Nelde, Annika, 72070 Tübingen (DE); Rammensee, Hans-Georg, 72070 Unterjesingen (DE); Bilich, Tatjana, 72072 Tübingen (DE)
(74) Representative: Witte, Weller & Partner Patentanwälte mbB

(57) **Abstract**

The present invention relates to peptides, proteins, nucleic acids and cells for use in immunotherapeutic methods. In particular, the present invention relates to the immunotherapy of an infection by SARS-CoV-2 (COVID-19). The present invention furthermore relates to SARS-CoV2-associated T-cell peptide epitopes that can for example serve as active pharmaceutical ingredients of vaccine compositions that stimulate anti-SARS-CoV2 immune responses, or to stimulate T cells ex *vivo* and transfer into patients. Peptides bound to molecules of the major histocompatibility complex (MHC), or peptides as such, can also be targets of antibodies, soluble T-cell receptors, and other binding molecules.

SEQUENCE LISTING

<110>  Eberhard-Karls-Universität, Medizinische Fakultät

<120>  Peptides and combinations of peptides for use in immunotherapy
       against an infection by SARS-CoV-2 (COVID-19)

<130>  5402P609EP

<160>  110

<170>  PatentIn version 3.5

<210>  1
<211>  10
<212>  PRT
<213>  SARS-CoV-2

<400>  1

Thr Thr Asp Pro Ser Phe Leu Gly Arg Tyr
1               5                   10

<210>  2
<211>  9
<212>  PRT
<213>  SARS-CoV-2

<400>  2

Leu Thr Asp Glu Met Ile Ala Gln Tyr
1               5

<210>  3
<211>  11
<212>  PRT
<213>  SARS-CoV-2

<400>  3

Ile Ser Glu His Asp Tyr Gln Ile Gly Gly Tyr
1               5                   10

<210>  4
<211>  9
<212>  PRT
<213>  SARS-CoV-2

<400>  4

Ala Gly Asp Ser Gly Phe Ala Ala Tyr
1               5

<210>  5
<211>  12
<212>  PRT
<213>  SARS-CoV-2

<400>  5

Arg Thr Phe Lys Val Ser Ile Trp Asn Leu Asp Tyr
1               5                   10

<210>  6
<211>  9
<212>  PRT
<213>  SARS-CoV-2

<400>  6

Arg Gln Glu Glu Val Gln Glu Leu Tyr
1               5

<210>  7
<211>  12
<212>  PRT
<213>  SARS-CoV-2

<400>  7

Val Asp Glu Ala Gly Ser Lys Ser Pro Ile Gln Tyr
1               5                   10

<210>  8
<211>  9
<212>  PRT
<213>  SARS-CoV-2

<400>  8

Ser Pro Asp Asp Gln Ile Gly Tyr Tyr
1               5

<210>  9
<211>  10
<212>  PRT
<213>  SARS-CoV-2

<400>  9

Gly Thr Gly Pro Glu Ala Gly Leu Pro Tyr
1               5                   10

<210>  10
<211>  10
<212>  PRT
<213>  SARS-CoV-2

<400>  10

Leu Ile Asp Leu Gln Glu Leu Gly Lys Tyr
1               5                   10

<210>  11
<211>  9
<212>  PRT

<213>  SARS-CoV-2

<400>  11

Phe Leu Leu Pro Ser Leu Ala Thr Val
1               5


<210>  12
<211>  9
<212>  PRT
<213>  SARS-CoV-2

<400>  12

Phe Ile Ala Gly Leu Ile Ala Ile Val
1               5


<210>  13
<211>  9
<212>  PRT
<213>  SARS-CoV-2

<400>  13

Ala Leu Ser Lys Gly Val His Phe Val
1               5


<210>  14
<211>  9
<212>  PRT
<213>  SARS-CoV-2

<400>  14

Phe Leu Ala Phe Val Val Phe Leu Leu
1               5


<210>  15
<211>  9
<212>  PRT
<213>  SARS-CoV-2

<400>  15

Lys Leu Leu Glu Gln Trp Asn Leu Val
1               5


<210>  16
<211>  11
<212>  PRT
<213>  SARS-CoV-2

<400>  16

Ser Ile Trp Asn Leu Asp Tyr Ile Ile Asn Leu
1               5                   10

<210>  17
<211>  10
<212>  PRT
<213>  SARS-CoV-2

<400>  17

Phe Leu Ile Val Ala Ala Ile Val Phe Ile
1               5                   10


<210>  18
<211>  9
<212>  PRT
<213>  SARS-CoV-2

<400>  18

Tyr Ile Asp Ile Gly Asn Tyr Thr Val
1               5


<210>  19
<211>  10
<212>  PRT
<213>  SARS-CoV-2

<400>  19

Leu Leu Leu Leu Asp Arg Leu Asn Gln Leu
1               5                   10


<210>  20
<211>  10
<212>  PRT
<213>  SARS-CoV-2

<400>  20

Val Leu Gln Leu Pro Gln Gly Thr Thr Leu
1               5                   10


<210>  21
<211>  9
<212>  PRT
<213>  SARS-CoV-2

<400>  21

Lys Leu Phe Ala Ala Glu Thr Leu Lys
1               5


<210>  22
<211>  9
<212>  PRT
<213>  SARS-CoV-2

<400>  22

Arg Leu Phe Arg Lys Ser Asn Leu Lys

1                   5


<210>  23
<211>  11
<212>  PRT
<213>  SARS-CoV-2

<400>  23

Arg Ile Phe Thr Ile Gly Thr Val Thr Leu Lys
1               5                   10


<210>  24
<211>  9
<212>  PRT
<213>  SARS-CoV-2

<400>  24

Asn Ile Val Asn Val Ser Leu Val Lys
1               5


<210>  25
<211>  9
<212>  PRT
<213>  SARS-CoV-2

<400>  25

Arg Ile Ala Gly His His Leu Gly Arg
1               5


<210>  26
<211>  9
<212>  PRT
<213>  SARS-CoV-2

<400>  26

Asn Leu Ile Ile Lys Asn Leu Ser Lys
1               5


<210>  27
<211>  10
<212>  PRT
<213>  SARS-CoV-2

<400>  27

Gln Leu Arg Ala Arg Ser Val Ser Pro Lys
1               5                   10


<210>  28
<211>  10
<212>  PRT
<213>  SARS-CoV-2

<400>  28

Lys Thr Phe Pro Pro Thr Glu Pro Lys Lys
1               5                   10


<210>  29
<211>  10
<212>  PRT
<213>  SARS-CoV-2

<400>  29

Lys Leu Asp Asp Lys Asp Pro Asn Phe Lys
1               5                   10


<210>  30
<211>  9
<212>  PRT
<213>  SARS-CoV-2

<400>  30

Val Thr Tyr Val Pro Ala Gln Glu Lys
1               5


<210>  31
<211>  9
<212>  PRT
<213>  SARS-CoV-2

<400>  31

Ala Ser Met Pro Thr Thr Ile Ala Lys
1               5


<210>  32
<211>  9
<212>  PRT
<213>  SARS-CoV-2

<400>  32

Ser Val Leu Asn Asp Ile Leu Ser Arg
1               5


<210>  33
<211>  9
<212>  PRT
<213>  SARS-CoV-2

<400>  33

Ala Ser Lys Ile Ile Thr Leu Lys Lys
1               5


<210>  34
<211>  10

<212>  PRT
<213>  SARS-CoV-2

<400>  34

Val Thr Leu Ala Ile Leu Thr Ala Leu Arg
1               5                   10


<210>  35
<211>  10
<212>  PRT
<213>  SARS-CoV-2

<400>  35

Gly Thr Ile Thr Val Glu Glu Leu Lys Lys
1               5                   10


<210>  36
<211>  9
<212>  PRT
<213>  SARS-CoV-2

<400>  36

Asn Leu Ile Ile Lys Asn Leu Ser Lys
1               5


<210>  37
<211>  9
<212>  PRT
<213>  SARS-CoV-2

<400>  37

Gly Val Lys His Val Tyr Gln Leu Arg
1               5


<210>  38
<211>  10
<212>  PRT
<213>  SARS-CoV-2

<400>  38

Ala Thr Glu Gly Ala Leu Asn Thr Pro Lys
1               5                   10


<210>  39
<211>  9
<212>  PRT
<213>  SARS-CoV-2

<400>  39

Ala Ser Ala Phe Phe Gly Met Ser Arg
1               5

<210>  40
<211>  9
<212>  PRT
<213>  SARS-CoV-2

<400>  40

Ser Ser Thr Ala Ser Ala Leu Gly Lys
1               5

<210>  41
<211>  9
<212>  PRT
<213>  SARS-CoV-2

<400>  41

Val Tyr Ile Gly Asp Pro Ala Gln Leu
1               5

<210>  42
<211>  9
<212>  PRT
<213>  SARS-CoV-2

<400>  42

Gln Tyr Ile Lys Trp Pro Trp Tyr Ile
1               5

<210>  43
<211>  9
<212>  PRT
<213>  SARS-CoV-2

<400>  43

Val Tyr Phe Leu Gln Ser Ile Asn Phe
1               5

<210>  44
<211>  10
<212>  PRT
<213>  SARS-CoV-2

<400>  44

Phe Tyr Val Tyr Ser Arg Val Lys Asn Leu
1               5                   10

<210>  45
<211>  10
<212>  PRT
<213>  SARS-CoV-2

<400>  45

Ser Tyr Phe Ile Ala Ser Phe Arg Leu Phe
1               5                   10

<210>  46
<211>  10
<212>  PRT
<213>  SARS-CoV-2

<400>  46

Pro Phe His Pro Leu Ala Asp Asn Lys Phe
1               5                   10

<210>  47
<211>  11
<212>  PRT
<213>  SARS-CoV-2

<400>  47

Glu Tyr His Asp Val Arg Val Val Leu Asp Phe
1               5                   10

<210>  48
<211>  11
<212>  PRT
<213>  SARS-CoV-2

<400>  48

Asp Tyr Lys His Trp Pro Gln Ile Ala Gln Phe
1               5                   10

<210>  49
<211>  10
<212>  PRT
<213>  SARS-CoV-2

<400>  49

Gly Tyr Ile Asn Val Phe Ala Phe Pro Phe
1               5                   10

<210>  50
<211>  11
<212>  PRT
<213>  SARS-CoV-2

<400>  50

Tyr Tyr Leu Gly Thr Gly Pro Glu Ala Gly Leu
1               5                   10

<210>  51
<211>  10
<212>  PRT
<213>  SARS-CoV-2

<400>  51

Ala Pro His Gly His Val Met Val Glu Leu
1               5                   10


<210>  52
<211>  9
<212>  PRT
<213>  SARS-CoV-2

<400>  52

Thr Pro Ile Asn Leu Val Arg Asp Leu
1               5


<210>  53
<211>  9
<212>  PRT
<213>  SARS-CoV-2

<400>  53

Ala Pro Phe Leu Tyr Leu Tyr Ala Leu
1               5


<210>  54
<211>  10
<212>  PRT
<213>  SARS-CoV-2

<400>  54

Lys Pro Ser Phe Tyr Val Tyr Ser Arg Val
1               5                   10


<210>  55
<211>  10
<212>  PRT
<213>  SARS-CoV-2

<400>  55

Arg Pro Leu Leu Glu Ser Glu Leu Val Ile
1               5                   10


<210>  56
<211>  10
<212>  PRT
<213>  SARS-CoV-2

<400>  56

His Pro Leu Ala Asp Asn Lys Phe Ala Leu
1               5                   10


<210>  57

<211>  9
<212>  PRT
<213>  SARS-CoV-2

<400>  57

Glu Pro Lys Leu Gly Ser Leu Val Val
1               5


<210>  58
<211>  9
<212>  PRT
<213>  SARS-CoV-2

<400>  58

Phe Pro Arg Gly Gln Gly Val Pro Ile
1               5


<210>  59
<211>  10
<212>  PRT
<213>  SARS-CoV-2

<400>  59

Phe Pro Phe Thr Ile Tyr Ser Leu Leu Leu
1               5                   10


<210>  60
<211>  10
<212>  PRT
<213>  SARS-CoV-2

<400>  60

Asn Pro Ala Asn Asn Ala Ala Ile Val Leu
1               5                   10


<210>  61
<211>  9
<212>  PRT
<213>  SARS-CoV-2

<400>  61

Tyr Leu Lys Leu Arg Ser Asp Val Leu
1               5


<210>  62
<211>  9
<212>  PRT
<213>  SARS-CoV-2

<400>  62

Glu Pro Val Leu Lys Gly Val Lys Leu
1               5

<210>  63
<211>  9
<212>  PRT
<213>  SARS-CoV-2

<400>  63

Ile Ile Lys Asn Leu Ser Lys Ser Leu
1               5


<210>  64
<211>  9
<212>  PRT
<213>  SARS-CoV-2

<400>  64

Thr Leu Asp Ser Lys Thr Gln Ser Leu
1               5


<210>  65
<211>  9
<212>  PRT
<213>  SARS-CoV-2

<400>  65

Thr Pro Lys Tyr Lys Phe Val Arg Ile
1               5


<210>  66
<211>  9
<212>  PRT
<213>  SARS-CoV-2

<400>  66

Val Pro Met Glu Lys Leu Lys Thr Leu
1               5


<210>  67
<211>  9
<212>  PRT
<213>  SARS-CoV-2

<400>  67

Phe Val Lys His Lys His Ala Phe Leu
1               5


<210>  68
<211>  9
<212>  PRT
<213>  SARS-CoV-2

<400>  68

Asp Leu Lys Gly Lys Tyr Val Gln Ile
1               5

<210>  69
<211>  9
<212>  PRT
<213>  SARS-CoV-2

<400>  69

Gly Ala Lys Leu Lys Ala Leu Asn Leu
1               5

<210>  70
<211>  9
<212>  PRT
<213>  SARS-CoV-2

<400>  70

Glu Ala Phe Glu Lys Met Val Ser Leu
1               5

<210>  71
<211>  9
<212>  PRT
<213>  SARS-CoV-2

<400>  71

Tyr Gln Lys Val Gly Met Gln Lys Tyr
1               5

<210>  72
<211>  9
<212>  PRT
<213>  SARS-CoV-2

<400>  72

Val Leu Lys Gly Val Lys Leu His Tyr
1               5

<210>  73
<211>  9
<212>  PRT
<213>  SARS-CoV-2

<400>  73

Phe Leu Tyr Leu Tyr Ala Leu Val Tyr
1               5

<210>  74
<211>  9
<212>  PRT

<213>  SARS-CoV-2

<400>  74

Leu Val Lys Pro Ser Phe Tyr Val Tyr
1               5


<210>  75
<211>  9
<212>  PRT
<213>  SARS-CoV-2

<400>  75

Trp Leu Ser Tyr Phe Ile Ala Ser Phe
1               5


<210>  76
<211>  9
<212>  PRT
<213>  SARS-CoV-2

<400>  76

Lys Val Ser Ile Trp Asn Leu Asp Tyr
1               5


<210>  77
<211>  9
<212>  PRT
<213>  SARS-CoV-2

<400>  77

Arg Gln Glu Glu Val Gln Glu Leu Tyr
1               5


<210>  78
<211>  9
<212>  PRT
<213>  SARS-CoV-2

<400>  78

Ile Gln Tyr Ile Asp Ile Gly Asn Tyr
1               5


<210>  79
<211>  9
<212>  PRT
<213>  SARS-CoV-2

<400>  79

Leu Leu Asn Lys His Ile Asp Ala Tyr
1               5

<210>  80
<211>  10
<212>  PRT
<213>  SARS-CoV-2

<400>  80

Asn Val Phe Ala Phe Pro Phe Thr Ile Tyr
1               5                   10


<210>  81
<211>  10
<212>  PRT
<213>  SARS-CoV-2

<400>  81

Ala Glu Ile Val Asp Thr Val Ser Ala Leu
1               5                   10


<210>  82
<211>  10
<212>  PRT
<213>  SARS-CoV-2

<400>  82

Ser Glu Pro Val Leu Lys Gly Val Lys Leu
1               5                   10


<210>  83
<211>  10
<212>  PRT
<213>  SARS-CoV-2

<400>  83

Ser Glu Leu Val Ile Gly Ala Val Ile Leu
1               5                   10


<210>  84
<211>  10
<212>  PRT
<213>  SARS-CoV-2

<400>  84

Tyr Glu Gly Asn Ser Pro Phe His Pro Leu
1               5                   10


<210>  85
<211>  10
<212>  PRT
<213>  SARS-CoV-2

<400>  85

Leu Glu Tyr His Asp Val Arg Val Val Leu

1                   5                        10


<210>  86
<211>  10
<212>  PRT
<213>  SARS-CoV-2

<400>  86

Met Glu Val Thr Pro Ser Gly Thr Trp Leu
1                   5                       10


<210>  87
<211>  10
<212>  PRT
<213>  SARS-CoV-2

<400>  87

Asn Glu Ser Leu Ile Asp Leu Gln Glu Leu
1                   5                       10


<210>  88
<211>  10
<212>  PRT
<213>  SARS-CoV-2

<400>  88

Thr Glu Ala Phe Glu Lys Met Val Ser Leu
1                   5                       10


<210>  89
<211>  10
<212>  PRT
<213>  SARS-CoV-2

<400>  89

Ile Glu Tyr Pro Ile Ile Gly Asp Glu Leu
1                   5                       10


<210>  90
<211>  10
<212>  PRT
<213>  SARS-CoV-2

<400>  90

Thr Glu Val Pro Ala Asn Ser Thr Val Leu
1                   5                       10


<210>  91
<211>  9
<212>  PRT
<213>  SARS-CoV-2

<400>  91

Asn Tyr Met Pro Tyr Phe Phe Thr Leu
1               5

<210>  92
<211>  9
<212>  PRT
<213>  SARS-CoV-2

<400>  92

Val Arg Phe Pro Asn Ile Thr Asn Leu
1               5

<210>  93
<211>  9
<212>  PRT
<213>  SARS-CoV-2

<400>  93

Tyr Tyr Gln Leu Tyr Ser Thr Gln Leu
1               5

<210>  94
<211>  9
<212>  PRT
<213>  SARS-CoV-2

<400>  94

Asn Arg Phe Leu Tyr Ile Ile Lys Leu
1               5

<210>  95
<211>  9
<212>  PRT
<213>  SARS-CoV-2

<400>  95

Ile Arg Gln Glu Glu Val Gln Glu Leu
1               5

<210>  96
<211>  9
<212>  PRT
<213>  SARS-CoV-2

<400>  96

Glu Tyr His Asp Val Arg Val Val Leu
1               5

<210>  97
<211>  9

<212>  PRT
<213>  SARS-CoV-2

<400>  97

Gln Arg Asn Ala Pro Arg Ile Thr Phe
1               5


<210>  98
<211>  9
<212>  PRT
<213>  SARS-CoV-2

<400>  98

Lys Lys Ala Asp Glu Thr Gln Ala Leu
1               5


<210>  99
<211>  9
<212>  PRT
<213>  SARS-CoV-2

<400>  99

Val Tyr Asp Pro Leu Gln Pro Glu Leu
1               5


<210>  100
<211>  9
<212>  PRT
<213>  SARS-CoV-2

<400>  100

Ile Tyr Asn Asp Lys Val Ala Gly Phe
1               5


<210>  101
<211>  15
<212>  PRT
<213>  SARS-CoV-2

<400>  101

Leu Asp Asp Phe Val Glu Ile Ile Lys Ser Gln Asp Leu Ser Val
1               5                   10                  15


<210>  102
<211>  15
<212>  PRT
<213>  SARS-CoV-2

<400>  102

Ile Thr Arg Phe Gln Thr Leu Leu Ala Leu His Arg Ser Tyr Leu
1               5                   10                  15

<210>  103
<211>  15
<212>  PRT
<213>  SARS-CoV-2

<400>  103

Phe Asn Gly Leu Thr Val Leu Pro Pro Leu Leu Thr Asp Glu Met
1               5                   10                  15


<210>  104
<211>  15
<212>  PRT
<213>  SARS-CoV-2

<400>  104

Phe Met Arg Ile Phe Thr Ile Gly Thr Val Thr Leu Lys Gln Gly
1               5                   10                  15


<210>  105
<211>  15
<212>  PRT
<213>  SARS-CoV-2

<400>  105

Phe Tyr Val Tyr Ser Arg Val Lys Asn Leu Asn Ser Ser Arg Val
1               5                   10                  15


<210>  106
<211>  15
<212>  PRT
<213>  SARS-CoV-2

<400>  106

Leu Ser Tyr Tyr Lys Leu Gly Ala Ser Gln Arg Val Ala Gly Asp
1               5                   10                  15


<210>  107
<211>  15
<212>  PRT
<213>  SARS-CoV-2

<400>  107

Ile Trp Asn Leu Asp Tyr Ile Ile Asn Leu Ile Ile Lys Asn Leu
1               5                   10                  15


<210>  108
<211>  15
<212>  PRT
<213>  SARS-CoV-2

<400>  108

Gln Glu Glu Val Gln Glu Leu Tyr Ser Pro Ile Phe Leu Ile Val
1               5                   10                  15

<210>  109
<211>  15
<212>  PRT
<213>  SARS-CoV-2

<400>  109

Ser Lys Trp Tyr Ile Arg Val Gly Ala Arg Lys Ser Ala Pro Leu
1               5                   10                  15

<210>  110
<211>  15
<212>  PRT
<213>  SARS-CoV-2

<400>  110

Ile Asn Val Phe Ala Phe Pro Phe Thr Ile Tyr Ser Leu Leu Leu
1               5                   10                  15

## Description

The present invention relates to peptides, proteins, nucleic acids and cells for use in immunotherapeutic methods. In particular, the present invention relates to the immunotherapy of an infection by SARS-CoV-2 (COVID-19). The present invention furthermore relates to SARS-CoV2-associated T-cell peptide epitopes that can for example serve as active pharmaceutical ingredients of vaccine compositions that stimulate anti-SARS-CoV2 immune responses, or to stimulate T cells *ex vivo* and transfer into patients. Peptides bound to molecules of the major histocompatibility complex (MHC), or peptides as such, can also be targets of antibodies, soluble T-cell receptors, and other binding molecules.

The present invention relates to several novel peptide sequences and their variants that can be used in vaccine compositions for eliciting anti-SARS-CoV2 immune responses, or as targets for the development of pharmaceutically/immunologically active compounds and cells.

### FIELD OF THE INVENTION

The present invention relates to the field of molecular biology, more particular to the field of molecular immunology.

### BACKGROUND OF THE INVENTION

The novel coronavirus SARS-CoV-2 is responsible for the COVID-19 lung disease, which especially in elderly, weakened and immunocompromised patients, shows severe and fatal courses. In the meantime, SARS-CoV-2 has spread to a worldwide pandemic with yet incalculable health, economic and socio-political consequences. So far, there are no established therapies and a vaccine is not yet available.

T-cell immunity plays an essential role in the control of viral infections. In particular, CD4+ T helper (Th) cells are essential for the regulation and maintenance of immune responses as well as for the production of anti-viral cytokines while cytotoxic CD8+ T cells (CTL) are responsible for the elimination of virus-infected cells. For the activation and function of T cells the recognition of viral antigens represented by short peptides presented by human leukocyte antigens (HLA) is indispensable. To decipher protective T-cell immune responses in the human population, an extensive identification and characterization of such viral-derived T-cell epitopes is therefore essential, followed by a detailed functional study of CD4+ and CD8+-specific T cells. Such knowledge is not only essential for the understanding of host immune defense and mechanisms of long-term protection upon virus rechallenge, but is also a prerequisite for developing new and more efficient immunotherapies.

It is, therefore, an object underlying the invention to provide SARS-CoV-2-derived T-cell epitopes which can be used to develop medicaments and therapeutic methods for the prophylaxis and treatment of an infection by SARS-CoV-2 (COVID-19) and which may allow a better understanding of the biology of SARS-CoV-2 and its transmission.

The present invention satisfies these and other needs.

### SUMMARY OF THE INVENTION

The present invention provides a peptide comprising an amino acid sequence selected from the group consisting of SEQ ID NO: 1 to SEQ ID NO: 110, and variant sequences thereof which are at least 88% homologous to SEQ ID NO: 1 to SEQ ID NO: 110, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

The inventors were able to predict 110 SARS-CoV-2-derived T-cell epitopes based on the established algorithms NetMHCpan and SYFPEITHI (www.syfpeithi.de). A total of 100 HLA class I and 10 HLA class II peptides from the ten described SARS-CoV-2 proteins are predicted, including ten peptides for each of the ten most common HLA class I and II allotypes. This will allow covering at least one HLA allotype for 91.7% of the world population and therefore will provide broadly applicable T-cell epitopes.

The term "peptide" is used herein to designate a series of amino acid residues, connected one to the other typically by peptide bonds between the alpha-amino and carbonyl groups of the adjacent amino acids. The peptides are preferably between 7 and 12 amino acids in length, further preferably between 8 and 11, but can be as long as 5, 6, 7, 8, 9, 10, 11, 12 or longer.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "peptide" shall also include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 15 amino acids in length.

The term "peptide" shall also include "polypeptide". The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an MHC molecule in substantially the same way as a peptide consisting of the given amino acid sequence in consisting of SEQ ID NO: 1 to SEQ ID NO: 110. For example, a peptide may be modified so that it at least maintains, if not improves, the ability to interact with and bind to the binding groove of a suitable MHC molecule, such as HLA-A*02, and in that way, it at least maintains, if not improves, the ability to bind to the TCR of activated T cells.

A person skilled in the art will be able to assess, whether T cells induced by a variant of a specific peptide will be able to cross-react with the peptide itself (Appay et al., 2006; Colombetti et al., 2006; Fong et al., 2001; Zaremba et al., 1997).

These T cells can subsequently cross-react with cells and kill cells that express a polypeptide that contains the natural amino acid sequence of the cognate peptide as defined in the aspects of the invention. As can be derived from the scientific literature and databases (Rammensee et al., 1999; Godkin et al., 1997), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. Thus, one skilled in the art would be able to modify the amino acid sequences set forth in SEQ ID NO: 1 to SEQ ID NO: 110, by maintaining the known anchor residues, and would be able to determine whether such variants maintain the ability to bind MHC class I or II molecules. The variants of the present invention retain the ability to bind to the TCR of activated T cells, which can subsequently cross-react with and kill cells that express a polypeptide containing the natural amino acid sequence of the cognate peptide as defined in the aspects of the invention.

In the present invention, the term "homologous" refers to the degree of identity between sequences of two amino acid sequences, i.e. peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each base or amino acid in the Reference Sequence that does not have a corresponding aligned base or amino acid in the Compared Sequence and
(ii) each gap in the Reference Sequence and
(iii) each aligned base or amino acid in the Reference Sequence that is different from an aligned base or amino acid in the Compared Sequence, constitutes a difference and (iiii) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

According to the invention "full-length polypeptide" refers to the source proteins from which the peptides are derived, e.g. SARS-CoV-2 encoded proteins, such as the 7096 amino acid (aa) long ORF1ab polyprotein (replicase complex), the 1273 aa long surface glycoprotein (S for spikes), the 75 aa long envelope protein (E), the 222 aa long membrane glycoprotein (M), the 419 aa long nucleocapsid phosphoprotein (N) and another five proteins (ORF3a, ORF6, ORF7a, ORF8 and ORF10).

The problem underlying the invention is herewith completely solved.

In an embodiment of the invention said peptide has the ability to bind to an MHC class-I or-II molecule, and wherein said peptide, when bound to said MHC, is capable of being recognized by CD4 and/or CD8 T cells.

This measure has the advantage that the capability of the peptide according to the invention to induce an immune response, in particular a T-cell response, is ensured.

In another embodiment of the invention the amino acid sequence thereof comprises a continuous stretch of amino acids according to any one of SEQ ID NO: 1 to SEQ ID NO: 110.

This measure has the advantage that the peptide according to the invention comprises all amino acids which are predicted as being involved in the induction of an immune response. The therapeutic efficacy is herewith further improved.

Another subject-matter of the present invention relates to an antibody, in particular a soluble or membrane-bound antibody, preferably a monoclonal antibody or fragment thereof, that specifically recognizes the peptide or variant thereof according to the invention, preferably when bound to an MHC molecule.

The term "antibody" or "antibodies" is used herein in a broad sense and includes both polyclonal and monoclonal antibodies. In addition to intact or "full" immunoglobulin molecules, also included in the term "antibodies" are fragments (e.g. CDRs, Fv, Fab and Fc fragments) or polymers of those immunoglobulin molecules and humanized versions of immunoglobulin molecules, as long as they exhibit any of the desired properties, i.e. specifically recognize the peptide or variant thereof according to the invention. Whenever possible, the antibodies of the invention may be purchased from commercial sources. The antibodies of the invention may also be generated using well-known methods.

The features, characteristics, advantages and embodiments disclosed for the peptide according to the invention apply to the antibody and fragment thereof correspondingly.

Another subject-matter of the invention relates to a T-cell receptor, preferably soluble or membrane-bound, or a fragment thereof, that is reactive with an HLA ligand, wherein said ligand is the peptide or variant thereof according to the invention, preferably when bound to an MHC molecule.

The term "T-cell receptor" (abbreviated TCR) according to the invention refers to a heterodimeric molecule comprising an alpha polypeptide chain (alpha chain) and a beta polypeptide chain (beta chain), wherein the heterodimeric receptor is capable of binding to a peptide antigen presented by an HLA molecule. The term also includes so-called gamma/delta TCRs.

The features, characteristics, advantages and embodiments disclosed for the peptide and antibody or fragment thereof according to the invention apply to the T-cell receptor correspondingly.

A still further subject-matter according to the invention relates to a nucleic acid, encoding for a peptide or variant thereof according to the invention, an antibody or fragment thereof according to the invention, a T-cell receptor or fragment thereof according to the invention, optionally linked to a heterologous promoter sequence, or an expression vector expressing said nucleic acid.

The nucleic acid coding for a particular peptide, oligopeptide, or polypeptide may be naturally occurring or they may be synthetically constructed. The nucleic acid (for example a polynucleotide) may be, for example, DNA, cDNA, PNA, RNA or combinations thereof, either single- and/or double- stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing a polypeptide according to the invention. As used herein the term "nucleic acid coding for (or encoding) a peptide" refers to a nucleotide sequence coding for the peptide including artificial (man-made) start and stop codons compatible for the biological system the sequence is to be expressed by, for example, a dendritic cell or another cell system useful for the production of TCRs. The term "promoter" means a region of DNA involved in binding of RNA polymerase to initiate transcription.

The features, characteristics, advantages and embodiments disclosed for the peptide according to the invention apply to the nucleic acid and expression vector correspondingly.

Another subject-matter of the invention relates to a recombinant host cell comprising the peptide according to the invention, the antibody or fragment thereof according to the invention, the T-cell receptor or fragment thereof according to the invention or the nucleic acid or the expression vector according to the invention, wherein said host cell preferably is selected from an antigen presenting cell, such as a dendritic cell, a T cell or an NK cell.

The features, characteristics, advantages and embodiments disclosed for the peptide according to the invention apply to the host cell correspondingly.

A still further subject-matter of the invention relates to an *in vitro* method for producing activated T lymphocytes, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to the invention.

The activated T cells that are directed against the peptides of the invention are useful in therapy. Thus, a further aspect of the invention provides activated T cells obtainable by the foregoing methods of the invention.

Activated T cells, which are produced by the above method, will selectively recognize a cell that aberrantly expresses a polypeptide that comprises an amino acid sequence of SEQ ID NO: 1 to SEQ ID NO: 110.

Another subject-matter according to the invention relates to a pharmaceutical composition comprising at least one active ingredient selected from the group consisting of the peptide according to the invention, the antibody or fragment thereof according to the invention, the T-cell receptor or fragment thereof according to the invention, the nucleic acid or the expression vector according to the invention, the recombinant host cell according to the invention, or the activated T lymphocyte according to the invention, or a conjugated or labelled active ingredient, and a pharmaceutically acceptable carrier, and optionally, pharmaceutically acceptable excipients and/or stabilizers.

A "pharmaceutical composition" is a composition suitable for administration to a human being in a medical setting. Preferably, a pharmaceutical composition is sterile and produced according to GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH2 group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

Preferably, the pharmaceutical composition of the present invention is an immunotherapeutic such as a vaccine. It may be administered directly into the patient, into the affected organ or systemically i.d., i.m., s.c., i.p. and i.v., or applied *ex vivo* to cells derived from the patient or a human cell line which are subsequently administered to the patient, or used *in vitro* to select a subpopulation of immune cells derived from the patient, which are then re-administered to the patient. If the nucleic acid is administered to cells *in vitro,* it may be useful for the cells to be transfected so as to co-express immune-stimulating cytokines, such as interleukin-2. The peptide may be substantially pure, or combined with an immune-stimulating adjuvant or used in combination with immune-stimulatory cytokines, or be administered with a suitable delivery system, for example liposomes. The peptide may also be conjugated to a suitable carrier such as keyhole limpet haemocyanin (KLH) or mannan (see WO 95/18145 and (Longenecker et al., 1993)). The peptide may also be tagged, may be a fusion protein, or may be a hybrid molecule. The peptides whose sequence is given in the present invention are expected to stimulate CD4 or CD8 T cells. However, stimulation of CD8 T cells is more efficient in the presence of help provided by CD4 T-helper cells. Thus, for MHC Class I epitopes that stimulate CD8 T cells the fusion partner or sections of a hybrid molecule suitably provide epitopes which stimulate CD4-positive T cells. CD4- and CD8-stimulating epitopes are well known in the art and include those identified in the present invention. In one aspect, the vaccine comprises at least one peptide having the amino acid sequence set forth SEQ ID NO: 1 to SEQ ID NO: 109, and at least one additional peptide, preferably two to 50, more preferably two to 25, even more preferably two to 20 and most preferably two, three, four, five, six, seven, eight, nine, ten, eleven, twelve, thirteen, fourteen, fifteen, sixteen, seventeen or eighteen peptides. The peptide(s) may be derived from one or more specific TAAs and may bind to MHC class I molecules.

In an embodiment of the pharmaceutical composition according to the invention it comprises at least 5, preferably at least 6, further preferably at least 7, further preferably at least 8, further preferably at least 9, and highly preferably at least 10 different peptides, each peptide comprising an amino acid sequence selected from the group consisting of SEQ ID NO: 1 to SEQ ID NO: 110, and variant sequences thereof which are at least 88% homologous to SEQ ID NO: 1 to SEQ ID NO: 110, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

The inventors have realized that a pharmaceutical composition, such as a vaccine, has a good immunogenic effect if at least 5 peptides or amino acid sequences out of the SEQ ID NOS. 1 to 110 are contained therein. When using 10 peptides or amino acid sequences 91.7% of the human world population may be covered. Preferably the vaccine comprises at peptides 1 to 9 peptides comprising amino acid sequences out of the SEQ ID NOS. 1 to 100, and 1 to 9 peptides comprising amino acid sequences out of the SEQ ID NOS. 101 to 110. "1 to 9" in this context means 1, 2, 3, 4, 5, 6, 7, 8, or 9.

In this context it is an preferred embodiment of the pharmaceutical composition if the at least 1 peptide or amino acid sequence is selected out of each of the following 'groups of ten': SEQ ID NOs: 1 to 10; SEQ ID NOs: 11 to 20; SEQ ID NOs: 21 to 30; SEQ ID NOs: 31 to 40; SEQ ID NOs: 41 to 50; SEQ ID NOs: 51 to 60; SEQ ID NOs: 61 to 70; SEQ ID NOs: 71 to 80; SEQ ID NOs: 81 to 90; SEQ ID NOs: 91 to 100 SEQ ID NOs: 101 to 110. "At least 1" means 1, 2, 3, 4, 5, 6, 7, 8, 9, 10. This ensures a coverage of 91.7% of the world population.

Another subject-matter of the invention relates to the peptide according to the invention, the antibody or fragment thereof according to the invention, the T-cell receptor or fragment thereof according to the invention, the nucleic acid or the expression vector according to the invention, the recombinant host cell according to the invention, or the activated T lymphocyte according to the invention for use in medicine, preferably for use against an infection by SARS-CoV-2 (COVID-19), such as a vaccine.

A still further subject-matter according to the invention relates to a kit comprising:
(a) a container comprising a pharmaceutical composition containing the peptide(s) or the variant according to the invention, the antibody or fragment thereof according to the invention, the T-cell receptor or fragment thereof according to the invention, the nucleic acid or the expression vector according to the invention, the recombinant host cell according to the invention, or the activated T lymphocyte according to the invention, in solution or in lyophilized form;
(b) optionally, a second container containing a diluent or reconstituting solution for the lyophilized formulation;
(c) optionally, at least one more peptide selected from the group consisting of SEQ ID NO: 1 to SEQ ID NO: 110, and
(d) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

Kits of the present invention preferably comprise a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

It is to be understood that the before-mentioned features and those to be mentioned in the following cannot only be used in the combination indicated in the respective case, but also in other combinations or in an isolated manner without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figure:

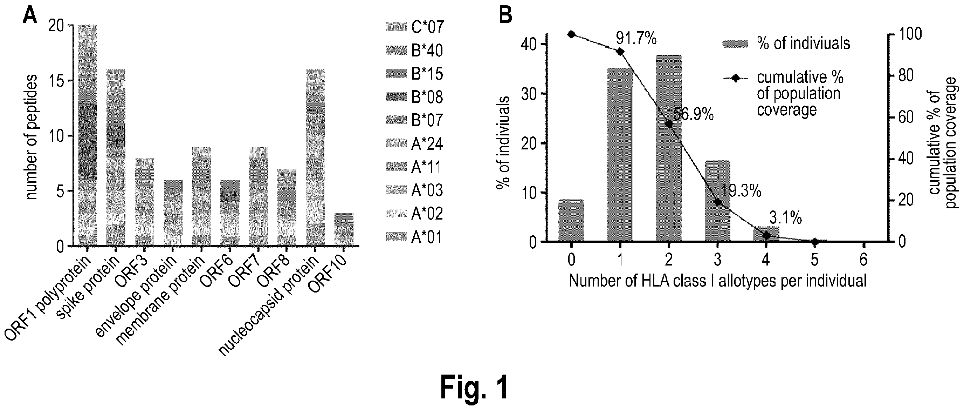

- Fig. 1: (A) Summary of predicted SARS-CoV-2 T-cell epitopes. The grey-scaled bars show the number of peptides predicted per allotype for the different SARS-Cov-2 proteins. (B) HLA class I allotype population coverage for the predicted SARS-CoV-2 peptide compared to the world population (calculated by the IEDB population coverage tool, www.iedb.org). The frequencies of individuals within the world population carrying up to six HLA allotypes (x-axis) of the ten allotypes used to predicted SARS-CoV-2 T-cell epitopes are indicated as grey bars on the left y-axis. The cumulative percentage of population coverage is depicted as black dots on the right y-axis.

### 1. General

The novel coronavirus SARS-CoV-2 is responsible for the COVID-19 lung disease, which especially in elderly, weakened and immunocompromised patients, shows severe and fatal courses. In the meantime, SARS-CoV-2 has spread to a worldwide pandemic with yet incalculable health, economic and socio-political consequences. So far, there are no established therapies and a vaccine is not yet available. Furthermore, it is not known whether and when T cell-mediated immunity against the novel SARS-CoV-2 virus is induced, let alone what the immunogenic epitopes of the virus are. Knowledge and experiences from two other zoonotic coronaviruses - SARS-CoV-1 and MERS-CoV - confirmed that T-cell immunity plays an important role in the recovery from coronavirus infections, with the detection of CoV-specific CD8+ and long-lasting memory CD4+ T-cell responses in convalescent individuals. Several CD4+ and CD8+ T-cell epitopes have been described for SARS-CoV-1 and MERS-CoV, which suggest due to the sequence homology of the two corona viruses potential cross reactivity between the two viruses and might also represent potential T-cell epitopes for the novel SARS-CoV-2 virus. However, most of the SARS-CoV-1 and MERS-CoV T-cell epitopes are so far limited to the spike protein and single HLA allotypes in particular HLA-A*02. Therefore, the an objective of the invention is the first-time characterization of SARS-CoV-2-specific CD4+ and CD8+ T-cell epitopes from all different proteins of the virus covering a wide range of the most common HLA allotypes. This approach will allow to i) gain more detailed knowledge about the interaction of SARS-CoV-2 with the immune system, ii) provide novel diagnostic tools, beside the detection of SARS-CoV-2-specific antibodies, which, from today's perspective, show a high cross-reactivity, to identify people with SARS-Cov-2 immunity and monitor T-cell immunity-based long-term protection, and furthermore iii) define possible target structures for the development of virus-specific immunotherapies for the treatment of the COVID-19 disease. Such therapeutic approaches include, for example, vaccination strategies and the adoptive transfer of virus-specific T cells or T-cell receptors (TCRs).

### 2. Data retrieval

The complete proteome sequence of SARS-CoV-2 isolate Wuhan-Hu-1 containing ten different open reading frames (ORFs) was retrieved from the NCBI database with the accession number MN908947.

### 3. Prediction of SARS-CoV-2-derived HLA class 1-binding peptides

The protein sequences of all ten ORFs were split into 9 - 12 amino acid long peptides covering the complete proteome of the virus. The prediction algorithms NetMHCpan 4.0 and SYFPEITHI 1.0 were used to predict the binding of the peptides to HLAA*01:01, A*02:01, A*03:01, A*11:01, A*24:02, B*07:02, B*08:01, B*15:01, B*40:01, and C*07:02. Only peptides predicted by both algorithms as HLA binding peptides (SYFPEITHI score ≥60%, NetMHCpan rank ≤2) for the respective allotype were further examined. Furthermore, peptides containing cysteines were excluded. The inventors ranked the peptides for each allotype and ORF separately according to their SYFPEITHI and NetMHCpan score, respectively. The final selection and ranking of the peptides was then based on the calculation of the average rank therefore combining NetMHCpan and SYFPEITHI-derived prediction scores in a single rank. The inventors then selected one peptide for each ORF and allotype aiming to receive 10 peptides in total for each allotype. From peptides with the same average rank, the inventors selected those with the higher SYFPEITHI score. For some allotypes not every ORF gave rise to an appropriate HLA-binding peptide. In those cases, the inventors filled up the remaining slots with additional peptides from the nucleocapsid protein, the spike protein, and the polyprotein ORF1. Finally, this selection process resulted in a list of 100 peptides for the ten most common HLA allotypes covering all different proteins of the virus (Table 1).

**Table 1: SARS-CoV-2-derived HLA class I-binding peptides**

| **SEQ ID** | **Immuno ID** | **Sequence** | **Protein** | **Protein name** | **ORF1 polyprotein region** | **HLA restriction** | **Syfpeithi Score** | **NetMHC Score** | **further allotypes** | **duplicate ID** |
|---|---|---|---|---|---|---|---|---|---|---|
| 1 | SARS_A01_P01 | TTDPSFLGRY | ORF1 | polyprotein | Papain-like proteinase | A01 | 89,7 | 0,00 | | |
| 2 | SARS_A01_P02 | LTDEMIAQY | ORF2 | spike protein | | A01 | 80,0 | 0,00 | | |
| 3 | SARS_A01_P03 | ISEHDYQIGGY | ORF3 | ORF3 | | A01 | 69,2 | 0,13 | | |
| 4 | SARS_A01_P04 | AGDSGFAAY | ORF5 | membrane protein | | A01 | 72,5 | 0,16 | | |
| 5 | SARS_A01_P05 | RTFKVSIWNLDY | ORF6 | ORF6 | | A01 | 65,9 | 1,57 | | |
| 6 | SARS_A01_P06 | RQEEVQELY | ORF7 | ORF7 | | A01 | 60,0 | 0,33 | B15 | 77 |
| 7 | SARS_A01_P07 | VDEAGSKSPIQY | ORF8 | ORF8 | | A01 | 73,2 | 1,23 | | |
| 8 | SARS_A01_P08 | SPDDQIGYY | ORF9 | nucleocapsid protein | | A01 | 67,5 | 0,23 | | |
| 9 | SARS_A01_P09 | GTGPEAGLPY | ORF9 | nucleocapsid protein | | A01 | 64,1 | 0,27 | | |
| 10 | SARS_A01_P10 | LIDLQELGKY | ORF2 | spike protein | | A01 | 74,4 | 0,09 | | |
| 11 | SARS_A02_P01 | FLLPSLATV | ORF1 | polyprotein | Non-structural protein 6 | A02 | 91,7 | 0,01 | | |
| 12 | SARS_A02_P02 | FIAGLIAIV | ORF2 | spike protein | | A02 | **83,3** | 0,13 | | |
| 13 | SARS_A02_P03 | ALSKGVHFV | ORF3 | ORF3 | | A02 | 80,6 | 0,04 | | |
| 14 | SARS_A02_P04 | FLAFVVFLL | ORF4 | envelope protein | | A02 | 72,2 | 0,30 | | |
| 15 | SARS_A02_P05 | KLLEQWNLV | ORF5 | membrane protein | | A02 | 72,2 | 0,06 | | |
| 16 | SARS_A02_P06 | SIWNLDYIINL | ORF6 | ORF6 | | A02 | **73,5** | 1,10 | | |
| 17 | SARS_A02_P07 | FLIVAAIVFI | ORF7 | ORF7 | | A02 | **79,4** | 1,33 | | |
| 18 | SARS_A02_P08 | YIDIGNYTV | ORF8 | ORF8 | | A02 | 69,4 | 0,03 | | |
| 19 | SARS_A02_P09 | LLLLDRLNQL | ORF9 | nucleocapsid protein | | A02 | **85,3** | 0,93 | | |
| 20 | SARS_A02_P10 | VLQLPQGTTL | ORF9 | nucleocapsid protein | | A02 | 67,7 | 1,07 | | |
| 21 | SARS_A03_P01 | KLFAAETLK | ORF1 | polyprotein | Helicase | A03 | 83,9 | 0,01 | | |
| 22 | SARS_A03_P02 | RLFRKSNLK | ORF2 | spike protein | | A03 | 87,1 | 0,01 | | |
| 23 | SARS_A03_P03 | RIFTIGTVTLK | ORF3 | ORF3 | | A03 | **81,3** | 0,14 | A11 | |
| 24 | SARS_A03_P04 | NIVNVSLVK | ORF4 | envelope protein | | A03 | 71,0 | 0,44 | A11 | |
| 25 | SARS_A03_P05 | RIAGHHLGR | ORF5 | membrane protein | | A03 | 74,2 | 0,08 | | |
| 26 | SARS_A03_P06 | NLIIKNLSK | ORF6 | ORF6 | | A03 | 77,4 | 0,28 | A11 | 36 |
| 27 | SARS_A03_P07 | QLRARSVSPK | ORF7 | ORF7 | | A03 | **67,7** | 0,78 | | |
| 28 | SARS_A03_P08 | KTFPPTEPKK | ORF9 | nucleocapsid protein | | A03 | **90,3** | 0,01 | A11 | |
| 29 | SARS_A03_P09 | KLDDKDPNFK | ORF9 | nucleocapsid protein | | A03 | 80,7 | 0,32 | | |
| 30 | SARS_A03_P10 | VTYVPAQEK | ORF2 | spike protein | | A03 | 71,0 | 0,02 | A11 | |
| 31 | SARS_A11_P01 | ASMPTTIAK | ORF1 | polyprotein | Papain-like proteinase | A11 | 82,4 | 0,00 | | |
| 32 | SARS_A11_P02 | SVLNDILSR | ORF2 | spike protein | | A11 | 79,4 | 0,06 | A03 | |
| 33 | SARS_A11_P03 | ASKIITLKK | ORF3 | ORF3 | | A11 | 79,4 | 0,11 | | |
| 34 | SARS_A11_P04 | VTLAILTALR | ORF4 | envelope protein | | A11 | **66,7** | 1,12 | | |
| 35 | SARS_A11_P05 | GTITVEELKK | ORF5 | membrane protein | | A11 | **87,9** | 0,18 | A03 | |
| 36 | SARS_A11_P06 | NLIIKNLSK | ORF6 | ORF6 | | A11 | 61,8 | 0,65 | A03 | 26 |
| 37 | SARS_A11_P07 | GVKHVYQLR | ORF7 | ORF7 | | A11 | **67,7** | 1,80 | | |
| 38 | SARS_A11_P08 | ATEGALNTPK | ORF9 | nucleocapsid protein | | A11 | **72,7** | 0,23 | | |
| 39 | SARS_A11_P09 | ASAFFGMSR | ORF9 | nucleocapsid protein | | A11 | 67,7 | 0,06 | | |
| 40 | SARS_A11_P10 | SSTASALGK | ORF2 | spike protein | | A11 | 85,3 | 0,10 | | |
| 41 | SARS_A24_P01 | VYIGDPAQL | ORF1 | polyprotein | Helicase | A24 | 80,7 | 0,03 | C07 | |
| 42 | SARS_A24_P02 | QYIKWPWYI | ORF2 | spike protein | | A24 | 77,4 | 0,03 | | |
| 43 | SARS_A24_P03 | VYFLQSINF | ORF3 | ORF3 | | A24 | 71,0 | 0,01 | C07 | |
| 44 | SARS_A24_P04 | FYVYSRVKNL | ORF4 | envelope protein | | A24 | **70,0** | 1,56 | | |
| 45 | SARS_A24_P05 | SYFIASFRLF | ORF5 | membrane protein | | A24 | 76,7 | 0,07 | | |
| 46 | SARS_A24_P06 | PFHPLADNKF | ORF7 | ORF7 | | A24 | 60,0 | 0,44 | | |
| 47 | SARS_A24_P07 | EYHDVRVVLDF | ORF8 | ORF8 | | A24 | **70,0** | 0,63 | | |
| 48 | SARS_A24_P08 | DYKHWPQIAQF | ORF9 | nucleocapsid protein | | A24 | 66,7 | 0,32 | | |
| 49 | SARS_A24_P09 | GYINVFAFPF | ORF10 | | | A24 | 76,7 | 0,24 | | |
| 50 | SARS_A24_P10 | YYLGTGPEAGL | ORF9 | nucleocapsid protein | | A24 | 73,3 | 0,63 | | |
| 51 | SARS_B07_P01 | APHGHVMVEL | ORF1 | polyprotein | Host translation inhibitor nsp1 | B07 | 86,7 | 0,04 | | |
| 52 | SARS_B07_P02 | TPINLVRDL | ORF2 | spike protein | | B07 | 63,6 | 0,15 | | |
| 53 | SARS_B07_P03 | APFLYLYAL | ORF3 | ORF3 | | B07 | 69,7 | 0,09 | | |
| 54 | SARS_B07_P04 | KPSFYVYSRV | ORF4 | envelope protein | | B07 | 63,3 | 1,87 | | |
| 55 | SARS_B07_P05 | RPLLESELVI | ORF5 | membrane protein | | B07 | **66,7** | 0,86 | | |
| 56 | SARS_B07_P06 | HPLADNKFAL | ORF7 | ORF7 | | B07 | 73,3 | 0,11 | | |
| 57 | SARS_B07_P07 | EPKLGSLVV | ORF8 | ORF8 | | B07 | 60,6 | 0,37 | | |
| 58 | SARS_B07_P08 | FPRGQGVPI | ORF9 | nucleocapsid protein | | B07 | 72,7 | 0,02 | | |
| 59 | SARS_B07_P09 | FPFTIYSLLL | ORF10 | | | B07 | **73,3** | 1,63 | | |
| 60 | SARS_B07_P10 | NPANNAAIVL | ORF9 | nucleocapsid protein | | B07 | 73,3 | 0,32 | | |
| 61 | SARS_B08_P01 | YLKLRSDVL | ORF1 | polyprotein | Non-structural protein 4 | B08 | 81,4 | 0,01 | | |
| 62 | SARS_B08_P02 | EPVLKGVKL | ORF2 | spike protein | | B08 | 69,8 | 0,17 | B07 | |
| 63 | SARS_B08_P03 | IIKNLSKSL | ORF6 | ORF6 | | B08 | 60,5 | 0,17 | | |
| 64 | SARS_B08_P04 | TLDSKTQSL | ORF2 | spike protein | | B08 | 60,5 | 0,19 | A02 | |
| 65 | SARS_B08_P05 | TPKYKFVRI | ORF1 | polyprotein | 3C-like proteinase | B08 | 79,1 | 0,02 | | |
| 66 | SARS_B08_P06 | VPMEKLKTL | ORF1 | polyprotein | Papain-like proteinase | B08 | 69,8 | 0,01 | B07 | |
| 67 | SARS_B08_P07 | FVKHKHAFL | ORF1 | polyprotein | Non-structural protein 6 | B08 | 72,1 | 0,02 | | |
| 68 | SARS_B08_P08 | DLKGKYVQI | ORF1 | polyprotein | Non-structural protein 10 | B08 | 76,7 | 0,04 | | |
| 69 | SARS_B08_P09 | GAKLKALNL | ORF1 | polyprotein | Non-structural protein 2 | B08 | 83,7 | 0,07 | | |
| 70 | SARS_B08_P10 | EAFEKMVSL | ORF1 | polyprotein | Non-structural protein 7 | B08 | 67,4 | 0,03 | | |
| 71 | SARS_B15_P01 | YQKVGMQKY | ORF1 | polyprotein | Helicase | B15 | 85,2 | 0,01 | | |
| 72 | SARS_B15_P02 | VLKGVKLHY | ORF2 | spike protein | | B15 | 88,9 | 0,04 | | |
| 73 | SARS_B15_P03 | FLYLYALVY | ORF3 | ORF3 | | B15 | **81,5** | 1,14 | A03 | |
| 74 | SARS_B15_P04 | LVKPSFYVY | ORF4 | envelope protein | | B15 | 77,8 | 0,09 | | |
| 75 | SARS_B15_P05 | WLSYFIASF | ORF5 | membrane protein | | B15 | 74,1 | 1,41 | | |
| 76 | SARS_B15_P06 | KVSIWNLDY | ORF6 | ORF6 | | B15 | 74,1 | 1,19 | A03 | |
| 77 | SARS_B15_P07 | RQEEVQELY | ORF7 | ORF7 | | B15 | 85,2 | 0,11 | A01 | 6 |
| 78 | SARS_B15_P08 | IQYIDIGNY | ORF8 | ORF8 | | B15 | 77,8 | 0,02 | | |
| 79 | SARS_B15_P09 | LLNKHIDAY | ORF9 | nucleocapsid protein | | B15 | 81,5 | 0,06 | | |
| 80 | SARS_B15_P10 | NVFAFPFTIY | ORF10 | | | B15 | 60,6 | 1,37 | | |
| 81 | SARS_B40_P01 | AEIVDTVSAL | ORF1 | polyprotein | Helicase | B40 | 71,9 | 0,02 | | |
| 82 | SARS_B40_P02 | SEPVLKGVKL | ORF2 | spike protein | | B40 | 90,6 | 0,29 | | |
| 83 | SARS_B40_P03 | SELVIGAVIL | ORF5 | membrane protein | | B40 | 87,5 | 0,12 | | |
| 84 | SARS_B40_P04 | YEGNSPFHPL | ORF7 | ORF7 | | B40 | 62,5 | 0,27 | | |
| 85 | SARS_B40_P05 | LEYHDVRVVL | ORF8 | ORF8 | | B40 | 90,6 | 0,11 | | |
| 86 | SARS_B40_P06 | MEVTPSGTWL | ORF9 | nucleocapsid protein | | B40 | 68,8 | 0,21 | | |
| 87 | SARS_B40_P07 | NESLIDLQEL | ORF2 | spike protein | | B40 | **71,9** | 0,50 | | |
| 88 | SARS_B40_P08 | TEAFEKMVSL | ORF1 | polyprotein | Non-structural protein 7 | B40 | 84,4 | 0,15 | | |
| 89 | SARS_B40_P09 | IEYPIIGDEL | ORF1 | polyprotein | Guanine-N7 methyltransferase | B40 | 71,9 | 0,06 | | |
| 90 | SARS_B40_P10 | TEVPANSTVL | ORF1 | polyprotein | Non-structural protein 10 | B40 | 75,0 | 0,10 | | |
| 91 | SARS_C07_P01 | NYMPYFFTL | ORF1 | polyprotein | Papain-like proteinase | C07 | 86,7 | 0,01 | A24 | |
| 92 | SARS_C07_P02 | VRFPNITNL | ORF2 | spike protein | | C07 | 76,7 | 0,00 | | |
| 93 | SARS_C07_P03 | YYQLYSTQL | ORF3 | ORF3 | | C07 | 73,3 | 0,05 | A24 | |
| 94 | SARS_C07_P04 | NRFLYIIKL | ORF5 | membrane protein | | C07 | 80,0 | 0,07 | | |
| 95 | SARS_C07_P05 | IRQEEVQEL | ORF7 | ORF7 | | C07 | 80,0 | 0,10 | | |
| 96 | SARS_C07_P06 | EYHDVRVVL | ORF8 | ORF8 | | C07 | 80,0 | 0,10 | A24 | |
| 97 | SARS_C07_P07 | QRNAPRITF | ORF9 | nucleocapsid protein | | C07 | 76,7 | 0,04 | | |
| 98 | SARS_C07_P08 | KKADETQAL | ORF9 | nucleocapsid protein | | C07 | 60,0 | 1,62 | | |
| 99 | SARS_C07_P09 | VYDPLQPEL | ORF2 | spike protein | | C07 | 76,7 | 0,08 | A24 | |
| 100 | SARS_C07_P10 | IYNDKVAGF | ORF1 | polyprotein | RNA-directed RNA polymerase | C07 | 80,0 | 0,02 | A24 | |

### 4. Prediction of SARS-CoV-2-derived HLA class II-binding peptides

For HLA class II predictions all ten ORFs were split into 15 amino acid long peptides. The prediction algorithm SYFPEITHI 1.0 was used to predict the binding to HLA DRB1*01:01, DRB1*03:01, DRB1*04:01, DRB1*07:01, DRB1*11:01, and DRB1*15:01. The 5% top-scoring peptides of each ORF (related to the total length of each ORF, 2% for ORF1) were selected and sorted according to their position within the protein. Peptide clusters containing different length variants around a common 9 amino acid long core sequence were preselected for each protein, respectively. Thereby, the inventors selected one cluster for each protein as well as two and ten clusters for the spike protein and the nucleocapsid protein, respectively. From these clusters the inventors chose those clusters for further analyses, which cover most different HLA-DR allotypes. From each selected cluster one representative peptide was produced as synthetic peptide, thereby avoiding peptides containing cysteines. Finally, this selection process resulted in a list of 10 promiscuous HLA-DR peptides covering all different proteins of the virus (Table 2).

**Table 2: SARS-CoV-2-derived HLA class II-binding peptides**

| **SEQ ID** | **Immuno-ID** | **Protein** | **Start Position** | **Sequence** |
|---|---|---|---|---|
| 101 | | ORF1 | 6751 | LDDFVEIIKSQDLSV |
| 102 | | ORF2 | 235 | ITRFQTLLALHRSYL |
| 103 | | ORF2 | 855 | FNGLTVLPPLLTDEM |
| 104 | | ORF3 | 4 | FMRIFTIGTVTLKQG |
| 105 | | ORF4 | 56 | FYVYSRVKNLNSSRV |
| 106 | | ORF5 | 176 | LSYYKLGASQRVAGD |
| 107 | | ORF6 | 26 | IWNLDYIINLIIKNL |
| 108 | | ORF7 | 90 | QEEVQELYSPIFLIV |
| 109 | | ORF8 | 43 | SKWYIRVGARKSAPL |
| 110 | | ORF10 | 4 | INVFAFPFTIYSLLL |

In the event of discrepancies between the sequences specified in Tables 1 and 2 and those specified in the sequence listing, the information in the sequence listing takes precedence and applies.

## Claims

1. A peptide comprising an amino acid sequence selected from the group consisting of SEQ ID NO: 1 to SEQ ID NO: 110, and variant sequences thereof which are at least 88% homologous to SEQ ID NO: 1 to SEQ ID NO: 110, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

2. The peptide according to claim 1, wherein said peptide has the ability to bind to an MHC class-I or-II molecule, and wherein said peptide, when bound to said MHC, is capable of being recognized by CD4 and/or CD8 T cells.

3. The peptide or variant thereof according to claim 1 or 2, wherein the amino acid sequence thereof comprises a continuous stretch of amino acids according to any one of SEQ ID NO: 1 to SEQ ID NO: 100.

4. An antibody, in particular a soluble or membrane-bound antibody, preferably a monoclonal antibody or fragment thereof, that specifically recognizes the peptide or variant thereof according to any of claims 1 to 3, preferably the peptide or variant thereof according to any of claims 1 to 3 when bound to an MHC molecule.

5. A T-cell receptor, preferably soluble or membrane-bound, or a fragment thereof, that is reactive with an HLA ligand, wherein said ligand is the peptide or variant thereof according to any of claims 1 to 3, preferably the peptide or variant thereof according to any of claims 1 to 3 when bound to an MHC molecule.

6. A nucleic acid, encoding for a peptide or variant thereof according to any one of claims 1 to 3, an antibody or fragment thereof according to claim 4, a T-cell receptor or fragment thereof according to claim 5, optionally linked to a heterologous promoter sequence, or an expression vector expressing said nucleic acid.

7. A recombinant host cell comprising the peptide according to any one of claims 1 to 3, the antibody or fragment thereof according to claim 4, the T-cell receptor or fragment thereof according to claim 5 or the nucleic acid or the expression vector according to claim 6, wherein said host cell preferably is selected from an antigen presenting cell, such as a dendritic cell, a T cell or an NK cell.

8. An *in vitro* method for producing activated T lymphocytes, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to any one of claims 1 to 3.

9. An activated T lymphocyte, produced by the method according to claim 8, that selectively recognizes a cell which presents a polypeptide comprising an amino acid sequence given in any one of claims 1 to 3.

10. A pharmaceutical composition comprising at least one active ingredient selected from the group consisting of the peptide according to any one of claims 1 to 3, the antibody or fragment thereof according to claim 4, the T-cell receptor or fragment thereof according to claim 5, the nucleic acid or the expression vector according to claim 6, the recombinant host cell according to claim 7, or the activated T lymphocyte according to claim 9, or a conjugated or labelled active ingredient, and a pharmaceutically acceptable carrier, and optionally, pharmaceutically acceptable excipients and/or stabilizers.

11. The pharmaceutical composition according to claim 10, wherein it comprises at least 5, preferably at least 6, further preferably at least 7, further preferably at least 8, further preferably at least 9, and highly preferably at least 10 different peptides, each peptide comprising an amino acid sequence selected from the group consisting of SEQ ID NO: 1 to SEQ ID NO: 110, and variant sequences thereof which are at least 88% homologous to SEQ ID NO: 1 to SEQ ID NO: 110, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

12. The peptide according to any one of claims 1 to 3, the antibody or fragment thereof according to claim 4, the T-cell receptor or fragment thereof according to claim 5, the nucleic acid or the expression vector according to claim 6, the recombinant host cell according to claim 7, or the activated T lymphocyte according to claim 9 for use in medicine.

13. The peptide according to claim 12, wherein the use is in diagnosis and/or prevention and/or treatment of an infection by SARS-CoV-2 (COVID-19), or for use in the manufacture of a medicament against an infection by SARS-CoV-2 (COVID-19).

14. The peptide according to claim 13, wherein said medicament is a vaccine.

15. A kit comprising:
(a) a container comprising a pharmaceutical composition containing the peptide(s) or the variant according to any one of claims 1 to 3, the antibody or fragment thereof according to claim 4, the T-cell receptor or fragment thereof according to claim 5, the nucleic acid or the expression vector according to claim 6, the recombinant host cell according to claim 7, or the activated T lymphocyte according to claim 9, in solution or in lyophilized form;
(b) optionally, a second container containing a diluent or reconstituting solution for the lyophilized formulation;
(c) optionally, at least one more peptide selected from the group consisting of SEQ ID NO: 1 to SEQ ID NO: 110, and
(d) optionally, instructions for (i) use of the solution or (ii) reconstitution and/or use of the lyophilized formulation.
